# EUROPEAN PATENT APPLICATION

(11) **EP 4 509 111 A1**
(43) Date of publication of application: **19.02.2025**
(21) Application number: 22958915.5
(22) Date of filing: 05.12.2022
(51) Int. Cl.: A61H 39/00, A61H 1/00, A61N 1/36, A61N 1/04

(54) **EMS EXERCISE DEVICE SYSTEM INCLUDING SENSOR**

(30) Priority: 15.09.2022 KR 20220116519
(71) Applicant: Ion International Co. Ltd, Hanam-si Gyeonggi-do 12925 (KR)
(72) Inventor: HAN, Jung Woo, Namyangju-si Gyeonggi-do 12284 (KR)
(74) Representative: Krauns, Christian
(86) International application number: PCT/KR2022/019610
(87) International publication number: WO 2024/058313

(57) **Abstract**

A sensor-equipped EMS exercise equipment system according to some embodiments of the present invention includes: an exercise equipment worn on a body of a user to apply low-frequency waves to the body of the user; a control box attached and detached from to the exercise equipment and transmitting a current to the exercise equipment; a detection module including a plurality of sensors and disposed in the exercise equipment to detect biometric information of the user; and an analysis module for analyzing the biometric information and controlling the control box and the exercise equipment based on analysis results, wherein the detection module includes a first sensor for detecting a skin temperature of the user, and the analysis module determines that a risk of burns is present and controls the control box when a value detected by the first sensor exceeds a preset first reference value.

## Description

### [Technical Field]

The present invention relates to a sensor-equipped EMS exercise equipment system, and more particularly, to a sensor-equipped EMS exercise equipment system for sensing a user using sensors equipped in an EMS exercise equipment, and analyzing sensing results to provide information suitable to a physical condition of the user.

### [Background Art]

Recently, as interest in physical appearance has increased, the number of people managing their bodies through personal training and the like has been increasing. In addition, due to the impact of the COVID-19 pandemic, the market for so-called home training equipment for exercising at home is increasing, and the development of related technologies is also actively occurring.

In addition, as the number of people looking for small exercise and massage appliances to exercise and massage regardless of time and place has increased, small exercise/massage appliances applicable to various sizes and sites have been released.

Among them, an electric muscle simulation (EMS) exercise equipment, which maximizes exercise effects by transmitting electrical stimulation to the user's body to cause muscle contraction and relaxation, has been used by many people.

The EMS exercise equipment is configured to use low-frequency micro-currents to stimulate muscles and generate exercise effects. The user may select several steps of frequency bands or microcurrents of various frequency bands may be randomly generated, so as to stimulate muscles.

However, this type of EMS exercise equipment applies low-frequency waves of a band selected by the user or low-frequency waves of a predetermined band to the user's body for a preset period of time. Due to over-currents and/or excessive exercise time settings unsuitable for the user's physical condition, there is a risk of burns to the skin or damage to the muscles.

In addition, because the low-frequency band or stimulation time suitable for the user's body information (such as muscle mass and body fat mass) may not be known, the user may arbitrarily select the exercise time and low-frequency band, and accordingly, it may be difficult to achieve optimal results.

### [Disclosure]

### [Technical Problem]

The present invention is disclosed to solve the above-mentioned conventional problem, and an object of the present invention is to provide a sensor-equipped EMS exercise equipment system for sensing a user using sensors equipped in an EMS exercise equipment, and analyzing sensing results to provide the user with information and notifications.

### [Technical Solution]

The present invention is implemented by a sensor-equipped EMS exercise equipment system including: an exercise equipment worn on a body of a user to apply low-frequency waves to the body of the user; a control box attached and detached from to the exercise equipment and transmitting a current to the exercise equipment; a detection module including a plurality of sensors and disposed in the exercise equipment to detect biometric information of the user; and an analysis module for analyzing the biometric information and controlling the control box and the exercise equipment based on analysis results, wherein the detection module includes a first sensor for detecting a skin temperature of the user, and the analysis module is configured to determine that a risk of burns is present and control the control box when a value detected by the first sensor exceeds a preset first reference value.

In addition, the exercise equipment may include: a wearing portion worn on the body of the user; and one or more pads disposed on the wearing portion and coming into close contact with the body of the user to apply the low-frequency waves to the body of the user, the detection module may include a second sensor for detecting a degree of contact between the one or more pads and the body of the user, and the analysis module may determine that a wearing condition of the wearing portion is incorrect when a value detected by the second sensor exceeds a preset second reference value.

In addition, the exercise equipment may further include a moisture supply device disposed on the wearing portion to supply moisture to each of the pads, the detection module may include a third sensor for measuring a heart rate of the user, and the analysis module may calculate a usage time of the exercise equipment based on times initially detected and last detected by the third sensor for the heart rate, and activate the moisture supply device when the calculated value exceeds a preset third reference value.

In addition, the moisture supply device may supply a preset amount of moisture to each of the pads when the heart rate is detected by the third sensor.

In addition, the detection module may include a fourth sensor for detecting muscle movements of the user, and the analysis module may determine that the muscle may be damaged when a muscle movement detection value detected by the fourth sensor exceeds a fourth reference value for a preset period of time.

In addition, the detection module may include a fifth sensor for measuring a body fat percentage and a muscle mass of the user the analysis module may provide information about exercise guide, information about dietary formula, and information about using the exercise equipment to the user, based on the body fat percentage and the muscle mass detected by the fifth sensor.

### [Advantageous Effects]

The sensor-equipped EMS exercise equipment system according to one embodiment of the present invention can appropriately control the EMS exercise equipment based on the user's body information, so that the user can use the EMS exercise equipment safely and conveniently.

In addition, the sensor-equipped EMS exercise equipment system according to one embodiment of the present invention can continuously provide the user with the user's body information, so that the user can easily check exercise effects and changes in the user's body information.

### [Description of Drawings]

FIG. 1 is a diagram for explaining a sensor-equipped EMS exercise equipment system according to one embodiment of the present invention;
FIG. 2 is a view for explaining an exercise equipment and a control box of the sensor-equipped EMS exercise equipment system according to one embodiment of the present invention;
FIG. 3 is a diagram for explaining the configuration of a detection module of the sensor-equipped EMS exercise equipment system according to one embodiment of the present invention;
FIG. 4 is a view for explaining a water supply device of the sensor-equipped EMS exercise equipment system according to one embodiment of the present invention; and
FIGS. 5 to 10 are diagrams for explaining analysis processes of an analysis module of the sensor-equipped EMS exercise equipment system according to one embodiment of the present invention.

### [Best Mode]

### [Mode for Invention]

The embodiments described in this specification and the configurations shown in the drawings are merely preferred examples according to the present disclosure, and there may be various modification that may replace the embodiments and the drawings of this specification at the time of filing of the present application.

The same reference numbers or symbols presented in each drawing of the present specification denote parts or components serving substantially the same function. Shapes, sizes and the like of the components in the drawings may be exaggerated for clearer description.

The terms used in this specification are merely used to describe the embodiments, and are not intended to limit and/or restrict the disclosed invention. The singular expression includes a plural expression unless the context clearly means otherwise. In this specification, it will be understood that the term such as "include" and "have" is intended to designate the presence of feature, number, step, operation, element, component, or a combination thereof recited in the specification, which does not preclude the possibility of the presence or addition of one or more other features, numbers, steps, operations, components, parts or combinations thereof.

The terms including ordinal numbers, such as "first," or "second", used in this specification may be used to describe various components, however, the components are not limited by the above terms. The terms are used only for the purpose of distinguishing one component from another component. For example, the first component may be referred to as the second component without departing from the scope of the present invention, and similarly, the second component may also be referred to as the first component.

Hereinafter, a sensor-equipped EMS exercise equipment system according to one embodiment of the present invention will be described with reference to the accompanying drawings.

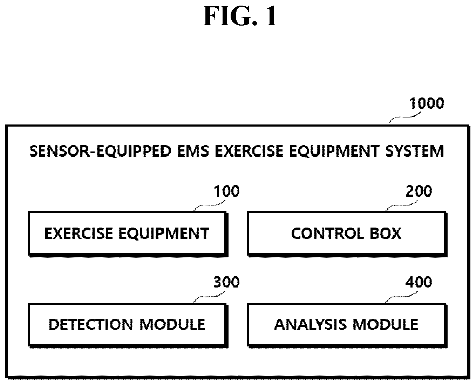

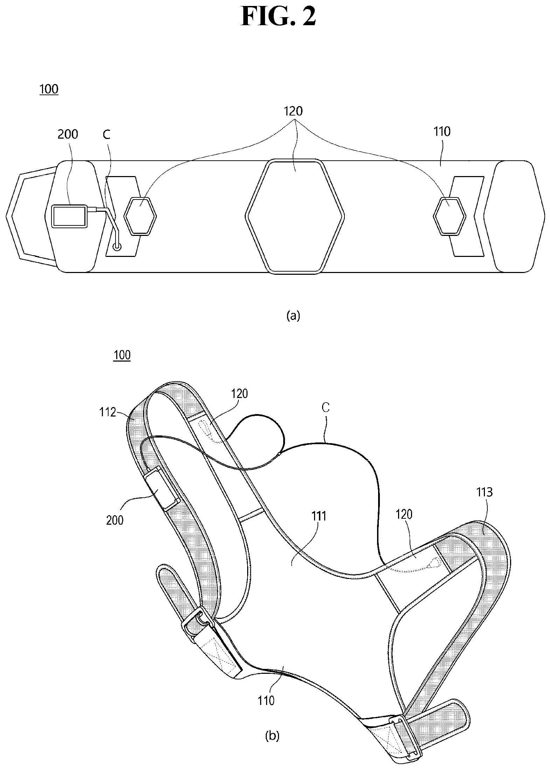

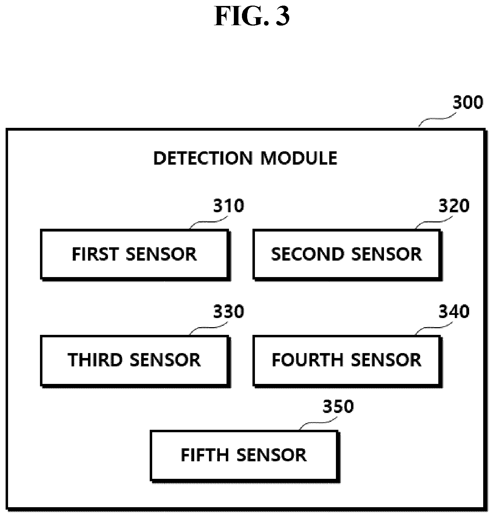

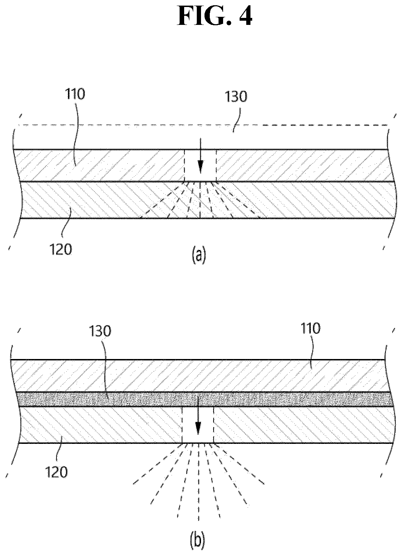

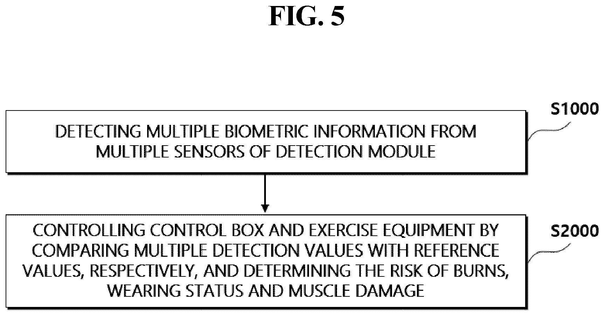

FIG. 1 is a diagram for explaining a sensor-equipped EMS exercise equipment system according to one embodiment of the present invention; FIG. 2 is a view for explaining an exercise equipment and a control box of the sensor-equipped EMS exercise equipment system according to one embodiment of the present invention; FIG. 3 is a diagram for explaining the configuration of a detection module of the sensor-equipped EMS exercise equipment system according to one embodiment of the present invention; FIG. 4 is a view for explaining a water supply device of the sensor-equipped EMS exercise equipment system according to one embodiment of the present invention; and FIGS. 5 to 10 are diagrams for explaining analysis processes of an analysis module of the sensor-equipped EMS exercise equipment system according to one embodiment of the present invention.

Referring to FIGS. 1 and 4, a sensor-equipped EMS exercise equipment system 1000 includes an exercise equipment 100, a control box 200, a detection module 300 and/or an analysis module 400.

The exercise equipment 100 is configured to be worn on the user's body to apply low-frequency waves to the user's body, and may be implemented in various shapes. More particularly, the exercise equipment 100 may include a wearing portion 110 and/or one or more pads 120.

The wearing portion 110 is configured to be worn directly on the user's body, and may be implemented in various shapes depending on a body part. For example, the wearing portion 110 may be implemented in the form of a belt shape designed to wrap around the user's waist or buttocks and/or a vest shape designed to wrap around the user's shoulders and back.

Referring to FIG. 2, the belt-shaped wearing portion 110 is provided to have a belt shape with a predetermined length, and may be worn by wrapping around the user's waist or buttocks. The belt-shaped wearing portion 110 may be implemented with a material having elasticity, and may be installed at both ends thereof with coupling members. The belt-shaped wearing portion 110 may have the coupling members installed at the both ends so as to be coupled to each other, thereby being worn and fixed on the user's waist or buttocks.

The vest-shaped wearing portion 110 may be implemented in a shape that wraps around the user's back and both shoulders. For example, the vest-shaped wearing portion 110 may include a rear portion 111 coming into contact with the user's upper back; and a first shoulder belt 112 and a second shoulder belt 113 each extending to have a predetermined length from an upper part of the rear portion 111, in which the first shoulder belt 112 and the second shoulder belt 113 may be worn to wrap around the user's shoulders. More specifically, the first shoulder belt 112 and the second shoulder belt 113 may be positioned at the user's chest area across the user's shoulders from the user's back, and ends of the first shoulder belt 112 and the second shoulder belt 113 may be connected to rings installed at lower ends of the rear portion 111, respectively. One side and an opposite side of each of the first shoulder belt 112 and the second shoulder belt 113 may be installed with Velcro tapes, respectively. In addition, each of the first shoulder belt 112 and the second shoulder belt 113 may be fixed by the Velcro tapes installed on the one side and the opposite side. The first shoulder belt 112 and the second shoulder belt 113 may have adjusted lengths by adjusting attached positions of the Velcro tapes. Accordingly, the wearing portion 110 may be adjusted to be suitable for a body shape of the user.

The wearing portion 110 may be installed therein with one or more pads 120 coming into close contact with the user's body to apply low-frequency waves to the user's body.

The one or more pads 120 are disposed on one side of the wearing portion 110, and may be disposed on a part coming into contact with the user's body when being worn by the user.

For example, an adhesive tool may be disposed on one side of the belt-shaped wearing portion 110, and the one or more pads 120 may be selectively attached to and detached from the one side of the wearing portion 110 by the adhesive tool. In other words, the one or more pads may be selectively attached to and detached from positions required by the user. The one or more pads may be a part coming into contact with the user's waist, buttocks, and/or abdomen when the user wears the wearing portion 110.

Meanwhile, the one or more pads 120 may be disposed at a position of the vest-shaped wearing portion 110 coming into contact with the user's shoulder part. More specifically, the one or more pads 120 may be disposed on each of the first shoulder belt 112 and the second shoulder belt 113. The one or more pads 120 may be one side coming into direct contact with the user's body when the user wears the wearing portion 110.

Meanwhile, the exercise equipment 100 may further include a moisture supply device 130. The moisture supply device 130 may be placed in the wearing portion 110 to supply moisture to each of the pads 120. More specifically, as shown in FIG. 4(a), the moisture supply device 130 may be disposed in the wearing portion 110 to receive moisture from a moisture storage case positioned outside the wearing portion 110. The moisture supply device 130 may include a moving portion for moving moisture supplied from the moisture storage case toward each of the pads 120, and a nozzle portion for spraying the moisture moved through the moving portion toward each of the pads 120. The moisture moved through the moving portion may be sprayed onto each of the pads 120 by the nozzle portion to supply the moisture to each of the pads 120. Accordingly, moisture can be automatically supplied to the pads 120 even when the user does not directly spray the moisture onto the pads 120.

Meanwhile, in the moisture supply device 130 as shown in FIG. 4(b), the moisture storage case for storing moisture may be disposed between the wearing portion 110 and each of the pads 120, and the moving portion may be disposed to pass through the pad 120. In addition, the nozzle portion may be installed at an end of the moving portion, and the moisture moved through the moving portion may be sprayed by the nozzle portion toward the outside of the pad 120. According to the configuration of the above moisture supply device 130, moisture is sprayed between the pad 120 and the user's body, so that the moisture can be automatically supplied between the pad 120 and the user's body. According to the present invention, adhesion between the user's body and the pad 120 may be improved, and low-frequency waves may be efficiently transmitted to the user's body.

When a heart rate is first detected by a third sensor 330 described later, the moisture supply device 130 may supply a preset amount of moisture to each of the pads 120. This will be described later.

The control box 200 may be attached to and detached from the exercise equipment 100, and may transmit a current to the exercise equipment 100. More specifically, the control box 200 may be selectively connected to the one or more pads 120 by a cable C.

The control box 200 may include a processor and a memory for operation of the exercise equipment 100. For example, the control box 200 may include a receiving circuit capable of receiving a user input. The user may manipulate the control box 200 to control the frequency, intensity, and/or applying time of a current applied to the exercise equipment 100. The control box 200 may further include a display, and the display may output information about operations of the exercise equipment 100 (such as operation time and operation mode).

Meanwhile, the control box 200 may be electrically connected to a terminal carried by the user and controlled through the terminal. For example, an application may be installed on a user terminal, and the user may control the control box 200 through actions, such as touching a screen, via the application. The control may refer to adjustment of the frequency, intensity, and/or applying time of the current applied to the exercise equipment 100.

Referring to FIG. 3, the detection module 300 may include a plurality of sensors, and may be disposed on the exercise equipment 100 to detect biometric information of the user. More particularly, the detection module 300 may include a first sensor 310, a second sensor 320, a third sensor 330, a fourth sensor 340 and/or a fifth sensor 350, and each of the sensors 310 to 350 may be provided in multiple units. The biometric information may include information about the user's skin temperature, information about the degree of contact between the one or more pads 120 and the user's body, information about the user's heart rate, information about the user's muscle movements and/or information about the user's body fat and muscle mass.

The first sensor 310 may be implemented as a temperature sensor for detecting the user's skin temperature, and may be disposed between the wearing portion 110 and the one or more pads 120 and/or on the one or more pads 120. In other words, the first sensor 310 may detect the user's skin temperature at a part coming into contact with the one or more pads 120. The first sensor 310 may operate when a current is applied to the one or more pads 120. The first sensor 310 may detect the user's skin temperature at the part coming into contact with the one or more pads 120, and transmit the detected value in real time to the analysis module 400 described later.

The second sensor 320 may be implemented as a distance detection sensor for detecting the degree of contact between the one or more pads 120 and the user's body. The second sensor 320 may be disposed between the wearing portion 110 and the one or more pads 120 and/or on the one or more pads 120. In other words, the second sensor 320 may detect the degree of adhesion by measuring the distance between the one or more pads 120 and the user's body coming into contact with the one or more pads 120.

The third sensor 330 may be implemented as a heart rate sensor for measuring the user's heart rate, and may be disposed on the wearing portion 110. The third sensor 330 may simultaneously measure the user's heart rate when the control box 200 applies a current to the one or more pads 120. In other words, the initial heart rate detection time of the third sensor 330 may be the same as the initial time when the low-frequency waves are applied to the user's body.

The fourth sensor 340 may be implemented as an electromyography sensor for detecting the user's muscle movements, and may be disposed between the wearing portion 110 and the one or more pads 120 and/or on the one or more pads 120. In other words, the fourth sensor 340 may detect muscle movements of the user's body part receiving the low-frequency waves applied from the one or more pads 120.

The fifth sensor 350 may be implemented as a sensor for measuring the user's body fat percentage and muscle mass, and may be disposed on the wearing portion 110. For example, when the user wears the wearing portion 110, the fifth sensor 350 may be disposed adjacent to the user's abdomen to measure the user's body fat percentage and muscle mass in the abdominal and waist parts. The fifth sensor 350 may selectively measure the body fat percentage and muscle mass by the user. In addition, the fifth sensor 350 may operate when the user actually wears the wearing portion 110 based on the user's settings. This will be described later.

Meanwhile, the sensors 310 to 350, most desirably, may be disposed in the above-described positions, but may be disposed in various places according to the shape of the wearing portion 110, and any position may be used as long as the purpose of detection by the sensors 310 to 350 is achieved. In addition, each detection value detected by each of the sensors 310 to 350 may be transmitted in real time to the analysis module 400.

According to another embodiment of the present invention, the detection module 300 of the sensor-equipped EMS exercise equipment system 1000 may further include a sixth sensor.

The sixth sensor may be implemented as a moisture detection sensor for detecting amounts of moisture in the one or more pads 120, and may be disposed on the one or more pads 120 to detect the amounts of moisture in the one or more pads 120.

The analysis module 400 may analyze biometric information detected by the detection module 300, and may control the control box 200 and the exercise equipment 100 based on the analysis results. More specifically, the analysis module 400 may analyze physical changes of the user using the exercise equipment 100 by comparing a plurality of detection values detected by the detection module 300 with a plurality of preset reference values, respectively.

The analysis module 400 may receive and analyze skin temperature detection values of the user detected by the detection module 300, detection values for the degree of contact between the one or more pads 120 and the user's body, a heart rate detection value of the user, a muscle movement detection value of the user, and/or body fat and muscle mass detection values of the user's body, and based on the analysis results, may provide an alarm to the user so that the user may use the exercise equipment 100 more efficiently. In addition, based on the analysis results, the analysis module 400 may control the control box 200 and the exercise equipment 100 in order to improve the effectiveness in use of the exercise equipment 100 by the user.

Meanwhile, the analysis module 400 may be connected to the terminal carried by the user to output the detection values detected by the detection module 300 and the analysis results to the user terminal. For example, an application may be installed on a user terminal, and the application may be connected to the exercise equipment 100, the control box 200, the detection module 300 and/or the analysis module 400. The application may output the detection values and the analysis results received from the components 100, 200, 300 and 400 in various forms on a terminal screen so as to be checked by the user. The output data and information may be in the form of text, two dimensional images, three dimensional images, graphs, tables, videos, audio and the like, and the period, time and each detection value to be output may be specified by the user's selection so that the detection values and the analysis results may be output in a limited manner.

FIGS. 5 to 10 are diagrams for explaining analysis processes of an analysis module of the sensor-equipped EMS exercise equipment system according to one embodiment of the present invention.

Referring to FIG. 5, the sensor-equipped EMS exercise equipment system 1000 according to one embodiment of the present invention may determine the user's risk of burns, wearing status, and/or muscle damage by using the analysis module 400.

In step S1000, each of the sensors 310 to 350 included in the detection module 300 may generate a detection value by sensing surroundings at the disposed position. The sensed detection value may include a skin temperature detection value of the user, a distance detection value between the one or more pads 120 and the user's body coming into contact with the one or more pads 120, a heart rate detection value of the user, a muscle movement detection value of the user, and/or a body fat percentage and muscle mass detection value of the user.

In step S2000, the analysis module 400 may determine the user's risk of burns, wearing status, and/or muscle damage by comparing the detection values with predefined reference value, respectively. In other words, the reference values may be preset to correspond to the multiple sensing items (temperature, distance, heart rate, muscle movement, body fat percentage, muscle mass) detected by the detection module 300, and the risk of burns, wearing status, and/or muscle damage of the user may be determined by comparing the detection values with the matching reference values.

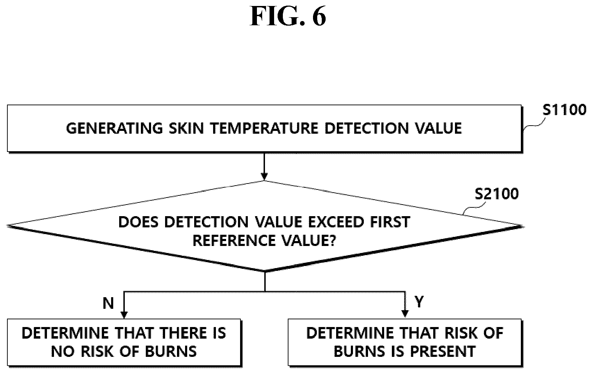

Referring to FIG. 6, the sensor-equipped EMS exercise equipment system 1000 according to one embodiment of the present invention may determine whether the user is at risk of burns.

In step S1100, the first sensor 310 of the detection module 300 may detect the user's skin temperature. The user's skin temperature refers to a temperature of the user's skin coming into contact with the one or more pads 120, and changes in the user's skin temperature due to low-frequency waves applied from the one or more pads 120 may be detected.

In step S2100, the analysis module 400 may compare the detected skin temperature detection value with a first reference value. The first reference value may be an initial skin temperature detection value of the user. The initial skin temperature detection value of the user refers to a skin temperature detection value first detected when the user wears the wearing portion 110, and may be preferable to be the skin temperature detection value before receiving the low-frequency waves applied from the one or more pads 120. The analysis module 400 may compare the detected skin temperature detection value with the first reference value, and determine that there is a risk of burns to the user's skin when the detected skin temperature detection value differs by 15% or more from the first reference value, and generate first risk information. The first risk information may include the skin temperature detection value differing by 15% or more from the first reference value and the user's low-frequency applying time.

Meanwhile, the analysis module 400 may also set a specific temperature as the first reference value. For example, the analysis module 400 may set the first reference value by a temperature at 45 degrees between 40degrees and 50 degrees, which is a temperature at which low-temperature burns may generally occur, and may determine that there is a risk of burns to the user's skin when the skin temperature detection value detected by the analysis module 400 is 45 degrees or higher, and generate first risk information. As the above description, the analysis module 400 may set the first reference value based on various data, and the first reference value may be selectively set by an operator.

In addition, the analysis module 400 may provide alarms to the user step by step and control the control box 200 when the user's skin temperature detection value continues a status of the set first reference value or higher. For example, when the user's skin temperature detection value remains at 45 degrees for 5 minutes or more, the analysis module 400 may transmit the first risk information to the user terminal, and lower intensity of the low-frequency waves applied to the user by controlling the control box 200. Thereafter, when the user's skin temperature detection value remains at 45 degrees for 5 minutes or more, the first risk information may be transmitted to the user terminal once again, and the control box 200 may be controlled to stop the low-frequency waves applied to the user. In other words, when the same risk information (here, the first risk information) is generated twice or more, the analysis module 400 may the control box 200 to completely stop the low-frequency waves applied to the user.

In addition, when the same risk information is generated twice or more, the analysis module 400 may control the moisture supply device 130. For example, when the same risk information is generated twice or more, the analysis module 400 may control the moisture supply device 130 so that moisture is supplied from the moisture supply device 130, thereby allowing the moisture to come into contact with the user's body. Accordingly, the user's skin temperature can decrease.

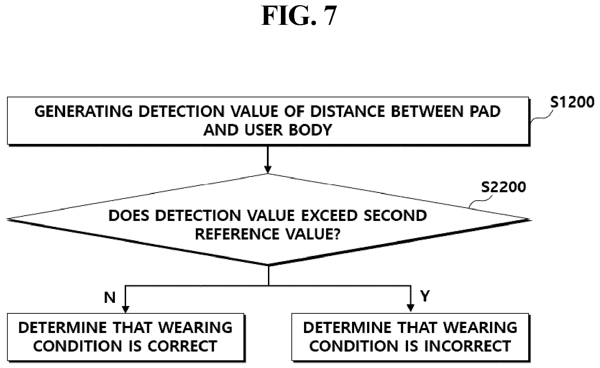

Referring to FIG. 7, the sensor-equipped EMS exercise equipment system 1000 according to one embodiment of the present invention may determine whether the user correctly wears the wearing portion 110.

In step S1100, the second sensor 320 of the detection module 300 may detect the degree of contact, that is a distance, between the one or more pads 120 and the user's body.

In step S2200, the analysis module 400 may compare the distance detection value with a second reference value. The second reference value may be 1 mm. This is because the low-frequency waves may be effectively applied to the body when the distance between the one or more pads 120 and the user's body is 0 mm, that is, when being in close contact with each other. When the detection value detected by the second sensor 320 exceeds the second reference value, the analysis module 400 may determine that the wearing state of the wearing portion 110 is incorrect and generate second risk information. The second risk information may be information about a detection value exceeding the second reference value. The second risk information may be transmitted to the user terminal to immediately notify the user that the wearing state of the wearing portion 110 is incorrect.

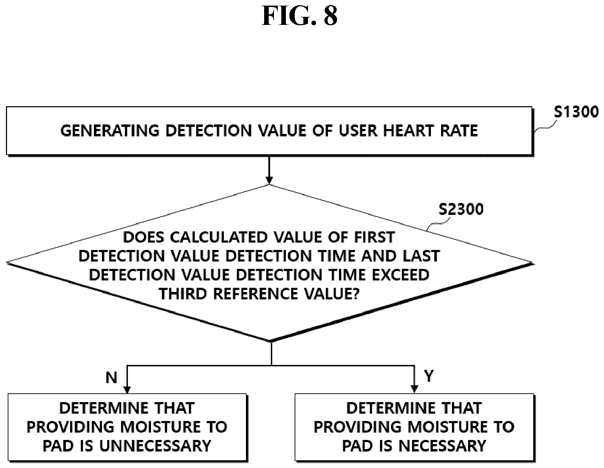

Referring to FIG. 8, the sensor-equipped EMS exercise equipment system 1000 according to one embodiment of the present invention may calculate actual usage time of the exercise equipment 100 by the user to supply moisture to the one or more pads 120.

In step S1100, the third sensor 330 of the detection module 300 may detect the user's heart rate. The third sensor 330 may be activated when being close at a preset reference value or less to the user's body. In other words, an operation may be performed at the moment the user actually wears the wearing portion 110 to detect the user's heart rate.

In step S2300, the analysis module 400 may analyze the user's usage time of the exercise equipment 100 by calculating the first time detected with the heart rate and the last time detected with the heart rate. Thereafter, when the analysis value, that is, the user's usage time of the exercise equipment 100 exceeds a third reference value, the analysis module 400 may control the moisture supply device 130 so that the moisture supply device 130 operates. The third reference value refers to time in which 50% or more of the moisture initially sprayed onto the one or more pads 120 evaporate, and may be set to be, for example, 20 minutes. The third reference value may be set by the user, and may be freely set depending on the situation.

In general, the exercise equipment 100 sprays the moisture onto the one or more pads 120 and then the wearing portion 110 is worn, so that the one or more pads 120 may come into close contact with the user's body. In addition, the low-frequency waves applied from the one or more pads 120 may be effectively transmitted to the user's body through the moisture sprayed onto the one or more pads 120. In other words, an appropriate amount of moisture is required to be present in the one or more pads 120 so as to efficiently transmit the low-frequency waves to the user's body. When moisture contents of the one or more pads 120 decreases, the conductivity may decrease, and accordingly, low-frequency waves having intensity lower than that of the actually applied low-frequency waves may be transmitted to the body. In other words, the low frequency applying efficiency may be significantly deteriorated. Accordingly, users have used the exercise equipment 100 by taking off the wearing portion 110 after a certain period of use, supplying moisture to the one or more pads 120 again, and then putting on the wearing portion 110.

However, when the sensor-equipped EMS exercise equipment system according to one embodiment of the present invention is used, the analysis module 400 may analyze the heart rate detected by the third sensor 330 to calculate the time the user actually has worn and used the exercise equipment 100, and control the moisture supply device 130 to allow moisture to be automatically sprayed from the moisture supply device 130 when the calculated value exceeds the third reference value, so that the inconvenience of taking off the exercise equipment 100 as time passes and spraying the moisture onto the one or more pads 120 can be resolved.

In addition, when the detection value calculated through the third sensor 330 exceeds the third reference value three times or more, that is, when usage duration time of the user's exercise equipment 100 is prolonged, the analysis module 400 may determine that the user is overusing the exercise equipment 100 and generate third risk information. The third risk information may include the user's actual usage time of the exercise equipment 100.

In addition, when the heart rate detected by the third sensor 330 is beyond the normal heart rate range, the analysis module 400 may determine that a risk situation has occurred for the user and generate the third risk information. The third risk information may include a heart rate beyond the normal heart rate range detected by the third sensor 330.

Meanwhile, when the initial heart rate is detected through the third sensor 330, the analysis module 400 may determine that the user is wearing the exercise equipment 100, and supply a preset amount of moisture to the one or more pads 120 through the moisture supply device 130. Accordingly, the inconvenience of the user required to spray moisture directly onto the one or more pads 120 before wearing the exercise equipment 100 can be resolved. In addition, only when the initial heart rate is detected by the third sensor 330 and simultaneously the detection value detected by the second sensor 320 is lower than the second reference value, the analysis module 400 may determine that the user is wearing the exercise equipment 100, and supply a preset amount of moisture to the one or more pads 120 through the moisture supply device 130. The above-described moisture supply conditions of the moisture supply device 130 may be selectively set by the user.

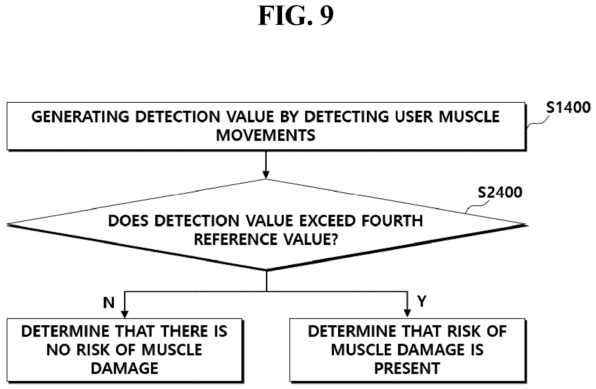

Referring to FIG. 9, the sensor-equipped EMS exercise equipment system 1000 according to one embodiment of the present invention may detect the user's muscle movements, thereby determining whether a risky situation causing damages to muscles has occurred.

In step S1100, the fourth sensor 340 of the detection module 300 may detect the user's muscle movements to generate a detection value. The fourth sensor 340 may be linked with the control box 200 to operate when the control box 200 starts to apply the low-frequency waves to the user. In other words, the fourth sensor 340 may detect the muscle movements when the low-frequency waves are applied to the user's body.

In step S2400, when the detection value detected by the fourth sensor 340 exceeds a fourth reference value, the analysis module 400 may determine that the muscle may be damaged. The fourth reference value may be a value beyond normal muscle movements. For example, when the number of repetitions in contraction and relaxation movements of the muscle detected by the fourth sensor 340 for 1 minute exceeds the fourth reference value, the analysis module 400 may determine that the user's muscle is moving in an abnormal pattern and generate fourth risk information. The fourth risk information may be a movement pattern of the user's muscle exceeding the fourth reference value. When the fourth risk information is generated, the analysis module 400 may adjust intensity of the low-frequency waves applied to the user by controlling the control box 200. In addition, when the same risk information (here, the fourth risk information) is generated twice or more, the analysis module 400 may control the control box 200 to stop the low-frequency waves applied to the user. In other words, the analysis module 400 may adjust intensity of the low-frequency waves to a lower level when the user's muscle is initially determined to be damaged, and may stop the low-frequency waves when the muscle is determined to be still damaged thereafter, so that the user's muscle may be gradually prevented from being damaged.

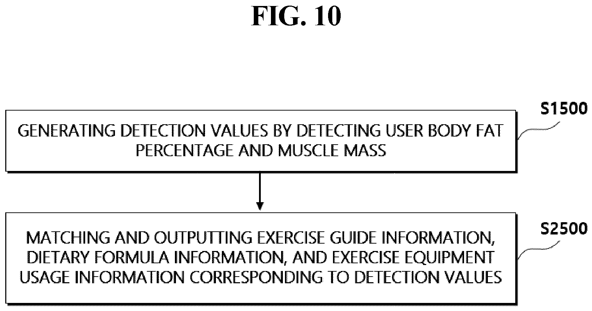

Referring to FIG. 10, the sensor-equipped EMS exercise equipment system 1000 according to one embodiment of the present invention may detect the user's body fat percentage and muscle mass, so that information about exercise guide, information about dietary formula, and information about using the exercise equipment 100, which are suitable for the user's body fat percentage and muscle mass, may be provided.

In step S1100, the fifth sensor 350 of the detection module 300 may detect the user's body fat percentage and muscle mass and generate corresponding detection values. The fifth sensor 350 may operate when the user wears the wearing portion 110. In other words, the fifth sensor 350 may operate when the user's heart rate is detected by the third sensor 330. In addition, the fifth sensor 350 may operate when the user's heart rate is detected by the third sensor 330 and simultaneously the detection value detected by the second sensor 320 is the second reference value or less, in order to detect the user's actual body fat percentage and muscle mass. The fifth sensor 350 may operate only when the user actually wears the exercise equipment 100 correctly on the body, so that the false detection rate may be significantly reduced.

In step S2500, based on the detection value detected by the fifth sensor 350, the analysis module 400 may provide information about exercise guide, information about dietary formula, and information about using the exercise equipment 100 suitable for the user. For example, the analysis module 400 may include a database, and the database may receive various information about exercise guide, information about dietary formula from an external server. In addition, the database may store information about using the exercise equipment 100 suitable for the body fat percentage and muscle mass.

The analysis module 400 may match and provide the information about exercise guide, the information about dietary formula, and the information about using the exercise equipment 100, which correspond to the body fat percentage and muscle mass detection values detected by the fifth sensor 350, to the user. The information about exercise guide, the information about dietary formula, and the information about using the exercise equipment may be categorized and stored according to the body fat percentage and muscle mass. For example, it may be divided into cases where the body fat percentage is 35% to 40%, cases where the body fat percentage is 30% to 34%, and cases where the body fat percentage is 25% to 30%, and the database may store exercise guide information including exercise types, exercise schemes and exercise times for each user's body fat percentage to become the average body fat percentage, dietary formula information including dietary formula types, dietary formula calories, dietary formula recipes, and dietary formula intake times, and/or exercise equipment 100 usage information including recommended frequency information and recommended frequency applying time information of the exercise equipment 100. The analysis module 400 may match various information corresponding to the body fat percentage detected by the fifth sensor 350 from a database and provided the matched information to the user. The analysis module 400 may provide the various information to the user through the user terminal. Accordingly, the user can obtain the exercise guide information, the dietary formula information, and the exercise equipment 100 usage information suitable for a current physical condition of the user.

The sensor-equipped EMS exercise equipment system 1000 according to another embodiment of the present invention may detect amounts of moisture contained in the one or more pads 120 to supply the moisture to the one or more pads 120.

The sixth sensor of the detection module 300 may detect the amounts of moisture contained in the one or more pads 120 to generate a detection value. The sixth sensor may be linked with the control box 200 to operate when the control box 200 starts to apply the low-frequency waves to the user. In other words, the sixth sensor may detect the amounts of moisture of the one or more pads 120 when the low-frequency waves are applied to the user's body.

Thereafter, when the detection value detected by the sixth sensor is a sixth reference value or less, the analysis module 400 may determine that moisture is insufficient in the one or more pads 120. The sixth reference value may be 50% of an initially detected moisture detection value of the one or more pads 120. When the moisture is determined to be insufficient in the one or more pads 120, the analysis module 400 may control the moisture supply device 130 to supply moisture to the one or more pads 120. The analysis module 400 may control the moisture supply device 130 to supply moisture to the one or more pads 120 until the one or more pads 120 have the initially detected moisture detection value.

The above-described sensor-equipped EMS exercise equipment system including the exercise equipment 100, the control box 200, the detection module 300 and the analysis module 400 can appropriately control the EMS exercise equipment based on the user's body information, so that the user can use the EMS exercise equipment safely and conveniently.

In addition, the user may be continuously provided with the user's body information, so that the user can easily check exercise effects and changes in the user's body information.

Although it has been described that all components constituting the embodiments of the present invention are combined as one or operated in combination, the present invention is not necessarily limited to the embodiments. In other words, one or more of all of the components may be selectively combined and operated within the scope of the present invention. In addition, Terms such as "include", "comprise" or "having" described above, signify that the corresponding component may be present therein unless specifically stated otherwise, so the above terms do not exclude other components, but may further include the other components. All terms including technical or scientific terms have the same meaning as generally understood by a person having ordinary skill in the art unless otherwise defined. Generally used terms, such as those defined in dictionaries, will be interpreted as consistent with the contextual meaning of the related art, and will not be interpreted as an ideal or excessively formal meaning unless expressly defined in the present invention.

In addition, the above descriptions are merely illustrative of the technical idea of the present invention, and it will be apparent that a person having ordinary skill in the art may carry out various deformations and modifications within the scope without departing from inherent features of the present invention. Accordingly, the embodiments disclosed in the present disclosure are intended to not limit but illustrate the technical idea of the present disclosure, so the scope of the technical idea of the present invention is not limited by those embodiments. The protection scope of the present invention will be understood according to the following claims, and all technical ideas within the scope of equivalents should be construed as falling within the scope of the present invention.

## Claims

1. A sensor-equipped EMS exercise equipment system comprising:
an exercise equipment worn on a body of a user to apply low-frequency waves to the body of the user;
a control box attached and detached from to the exercise equipment and transmitting a current to the exercise equipment;
a detection module including a plurality of sensors and disposed in the exercise equipment to detect biometric information of the user; and
an analysis module for analyzing the biometric information and controlling the control box and the exercise equipment based on analysis results, wherein
the detection module includes a first sensor for detecting a skin temperature of the user, and
the analysis module is configured to determine that a risk of burns occurs and control the control box when a value detected by the first sensor exceeds a preset first reference value.

2. The sensor-equipped EMS exercise equipment system of claim 1, wherein the exercise equipment includes:
a wearing portion worn on the body of the user; and
one or more pads disposed on the wearing portion and coming into close contact with the body of the user to apply the low-frequency waves to the body of the user, wherein
the detection module includes a second sensor for detecting a degree of contact between the one or more pads and the body of the user, and
the analysis module determines that a wearing condition of the wearing portion is incorrect when a value detected by the second sensor exceeds a preset second reference value.

3. The sensor-equipped EMS exercise equipment system of claim 2, wherein the exercise equipment further include:
a moisture supply device disposed on the wearing portion to supply moisture to each of the pads, wherein
the detection module includes a third sensor for measuring a heart rate of the user, and the analysis module calculates a usage time of the exercise equipment based on times initially detected and last detected by the third sensor for the heart rate to activate the moisture supply device when the calculated value exceeds a preset third reference value.

4. The sensor-equipped EMS exercise equipment system of claim 3, wherein the moisture supply device supplies a preset amount of moisture to each of the pads when the heart rate is detected by the third sensor.

5. The sensor-equipped EMS exercise equipment system of claim 1, wherein the detection module includes a fourth sensor for detecting muscle movements of the user, and
the analysis module determines that muscles have a risk of damage when a muscle movement detection value detected by the fourth sensor exceeds a fourth reference value for a preset period of time.

6. The sensor-equipped EMS exercise equipment system of claim 1, wherein the detection module includes a fifth sensor for measuring a body fat percentage and a muscle mass of the user, and
the analysis module provides the user with exercise guide information, dietary formula information, and exercise equipment usage information based on the body fat percentage and the muscle mass detected by the fifth sensor.
